# EUROPEAN PATENT SPECIFICATION

(11) **EP 2 227 140 B1**
(45) Date of publication and mention of the grant of the patent: **13.07.2016**
(21) Application number: 08848503.2
(22) Date of filing: 04.11.2008
(51) Int. Cl.: A61B 5/05, H01Q 13/10, H01Q 3/24, H01Q 21/06

(54) **ANTENNA FOR INVESTIGATING STRUCTURE OF HUMAN OR ANIMAL**
ANTENNE ZUR UNTERSUCHUNG DER STRUKTUR EINES MENSCHEN ODER TIERES
ANTENNE POUR EXAMINER UNE STRUCTURE D'HUMAIN OU D'ANIMAL

(30) Priority: 05.11.2007 GB 0721693
(43) Date of publication of application: 15.09.2010
(73) Proprietor: Micrima Limited, Bristol BS1 6EG (GB)
(72) Inventor: CRADDOCK, Ian, James, Bristol BS9 3UG (GB); KLEMM, Maciej, Bartlomiej, Bristol BS16 7EG (GB); GIBBONS, David, Rhys, Bristol BS6 6HR (GB)
(74) Representative: Haseltine Lake LLP
(86) International application number: PCT/GB2008/003718
(87) International publication number: WO 2009/060181

(56) References cited:
- EP-A- 0 527 417
- WO-A-2005/031919
- GB-A- 2 428 093

## Description

### FIELD OF THE INVENTION

The present invention relates to a system and method for investigating the internal structure of a human or animal body, by transmitting electromagnetic energy into the body from one or more antennas, and detecting the effect of the body on the passage of the electromagnetic energy. Each antenna comprising a slot formed in a conductive element.

### BACKGROUND OF THE INVENTION

An eccentric annular slot antenna for breast cancer detection is described in "Eccentric annular slot antenna for breast cancer detection based on the finite-difference-time-domain method" Raja, V.K. El-Shenawee, M., Wireless Communications and Applied Computational Electromagnetics, 2005. IEEE/ACES International Conference, Publication Date: 3-7 April 2005, page(s): 401- 404, ISBN: 0-7803-9068-7.

The antenna comprises an annular slot having an external boundary defined by a circular internal edge of a conductive sheet, and an internal boundary defined by an "island" of material offset from the centre of the external boundary.

This antenna suffers from a number of problems. Firstly, the slot has a relatively complex geometry, requiring the "island" of material to be mounted in a precise position. Secondly, the circular internal edge must be relatively large to physically accommodate the "island", and as a result the dimensions of the antenna (in the plane of the sheet) must be relatively large. The circular external boundary of the slot is 3cm across and the sheet of material is approximately 5cm across. Thirdly, the bandwidth of the slot at -10dB is relatively low ("almost 3.87 GHz").

Another antenna for breast cancer detection is described in AN ULTRA WIDEBAND MICROWAVE IMAGING SYSTEM FOR BREAST CANCER DETECTION, Wee Chang Khor and Marek E. Bialkowski, 10th Australian Symposium on Antennas, Sydney, Australia, 14-15 Feb. 2007.

In this case the antenna is a tapered slot antenna operating between 3.1 GHz and 10 GHz. The antenna is even larger than the antenna described in Raja et al - in this case approximately 6cm across.

Another antenna for breast cancer detection is described in "Dielectric-filled slotline bowtie antenna for breast cancer detection" by Shannon, C.J.; Fear, E.C.; Okoniewski, M., Electronics Letters, Volume 41, Issue 7, 31 March 2005 Page(s): 388 - 390, this is again a tapered slot measuring many cm in size.

A wide-slot antenna is described in Jia-Yi Sze, and Kin-Lu Wong, "Bandwidth enhancement of a microstrip-line-fed printed wide-slot antenna," IEEE Transactions on Antennas and Propagation, Volume 49, July 2001 pp: 1020 - 1024 (referred to below as "Sze et al"). The antenna is a square, wide slot antenna with a dual fork-shaped, microstrip feed built on a substrate with relative permittivity of 4.4. It is not designed for breast cancer detection, but is instead intended to radiate into free-space with an operating bandwidth of 4.5GHz from 2 - 6.5GHz.

GB-A-2428093 describes an instrument to non-invasively measure information concerning biological systems using low power sources emitting energy in the microwave region of the electromagnetic spectrum.

In WO 2005/031919 A a broadband slot array antenna employs a closely arranged array of slot antennas to minimize antenna size. The antenna array includes a common input terminal; a conductor plate having a common slot formed in a predetermined area and a plurality of slot halves being formed separately and respectively communicating with the common slot via a plurality of slot necks spaced by a predetermined distance; a plurality of feed lines, each having one terminus connected to the common input terminal, for applying power to the conductor plate at a cross coupling point; and a dielectric layer disposed between the conductor plate and the plurality of feed lines.

In EP-A-0527417 an antenna is designed to operate well below resonance, and includes a small flat cavity on the surface of which is made at least one radiating slot of much smaller dimensions than those of a normally resonating slot.

An object of the invention is to provide an antenna which is suitable for investigating the internal structure of a human or animal body, whilst being small and having a high bandwidth.

### SUMMARY OF THE INVENTION

A first aspect of the invention provides a system for investigating the internal structure of a human or animal body, the system comprising:
one or more antennas for transmitting and/or receiving electromagnetic energy into or from the body, each antenna comprising a slot formed in a conductive element,
the slot having an external boundary defined by a substantially closed internal edge of the conductive element;
a receiver configured to detect the effect of the body on the passage of the electromagnetic energy by recording one or more signals; and
a processor configured to process the signal(s) in order to generate an output indicative of the internal structure of the body,
characterized in that the antenna slot is a continuous slot with no internal boundary, the boundary of the slot being completely defined by the internal edge of the conductive element, and in that the slot of each antenna has a square or rectangular shape and a maximum dimension which is smaller than 30mm.

In contrast with the slot in Raja et al. which has an internal boundary, the slot is a continuous slot with no internal boundary. In contrast with the antenna in Khor et al., and the antenna in Shannon et al the slot has an external boundary defined by a substantially closed internal edge of the conductive element. Note that the boundary need not be completely closed, that is it may be substantially closed but with a small opening.

Typically each antenna further comprises one or more conductive elements for coupling energy to and/or from the slot. The conductive element may be for example a patch, a feed line, or two or more conductive feed lines, each feed line coupling with a respective part of the slot.

The use of two or more conductive feed lines provides a relatively high bandwidth. In this case the slot may comprise an open slot, an annular slot, or a continuous slot with no internal boundary, the external boundary of the slot being completely defined by the internal edge of the conductive element.

Typically each antenna is configured to transmit and/or receive radiation in a range of wavelengths including a minimum, maximum and centre wavelength, and the slot of each antenna has a maximum dimension which is smaller than the centre wavelength.

The slot of each antenna has a maximum dimension which is smaller than 30mm, and preferably smaller than 18mm.

The system may comprise only a single antenna which is used both to transmit electromagnetic energy into the body, and also acts as part of the receiver to detect the effect of the body on the passage of the electromagnetic energy. However more preferably the system comprises two or more antennas. In this case some of the antennas may be dedicated transmitters and others may be dedicated receivers, or alternatively the antennas may act either sequentially or simultaneously as a transmitter and a receiver. In a preferred mode of operation the antennas are driven sequentially, and the remaining (non-transmitting) antennas act as receivers.

The slot may be square (as in Sze et al) or it may have an elongated rectangular shape.

The slot may be planar or non-planar. For instance, the antenna may be conformed to a surface of a human or animal body.

The conducting element in which the slot is formed may comprise a simple thin sheet of a conducting material or alternatively it may comprise a layer of conducting material on a printed circuit board or suitable microwave substrate.

A further aspect of the invention provides a method of investigating the internal structure of a human or animal body with the system of the first aspect, the method comprising:
transmitting and/or receiving electromagnetic energy into and/or from the body with the antenna(s);
detecting the effect of the body on the passage of the electromagnetic energy by recording one or more signals; and
processing the signal(s) in order to generate an output indicative of the internal structure of the body.

Preferably the method is used to investigate the internal structure of a human breast.

### BRIEF DESCRIPTION OF THE DRAWINGS

Embodiments of the invention will now be described with reference to the accompanying drawings, in which:
Figure 1A is a plan view of a first antenna;
Figure 1B is a side view of the antenna of Figure 1A;
Figure 1C is a cross-sectional side view of a variant of the antenna of Figure 1A;
Figure 2 is a plan view of a second antenna;
Figure 3 is a graph showing simulated s₁₁ for slots with 17mm *x*-dimension and varying *z-*dimension;
Figure 4 is a graph showing simulated bore sight transfer functions at 35mm into the phantom for the antenna configurations with varying ground plane sizes;
Figure 5 is a graph showing simulated s11 for antennas with a 17x12mm slot, 1x5mm ground plane and various feed gaps; and
Figure 6 is a schematic view of a system for investigating the internal structure of a human breast.

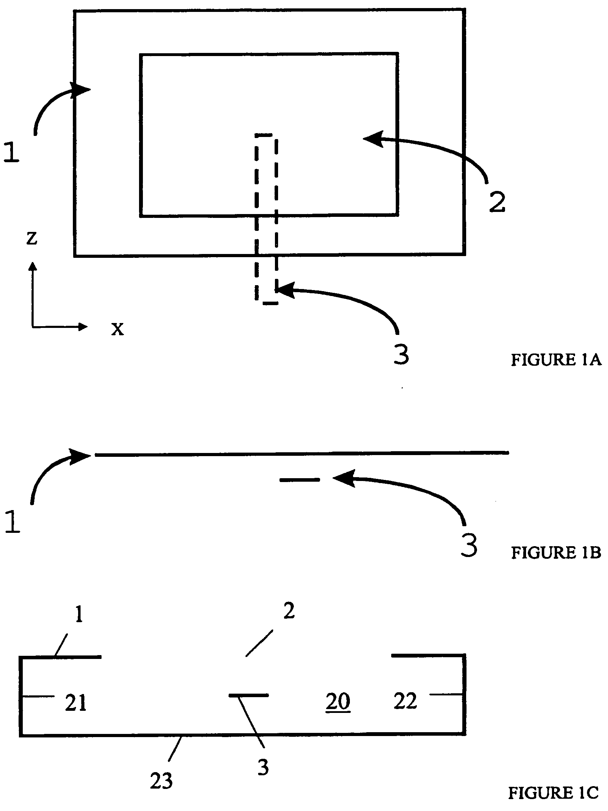

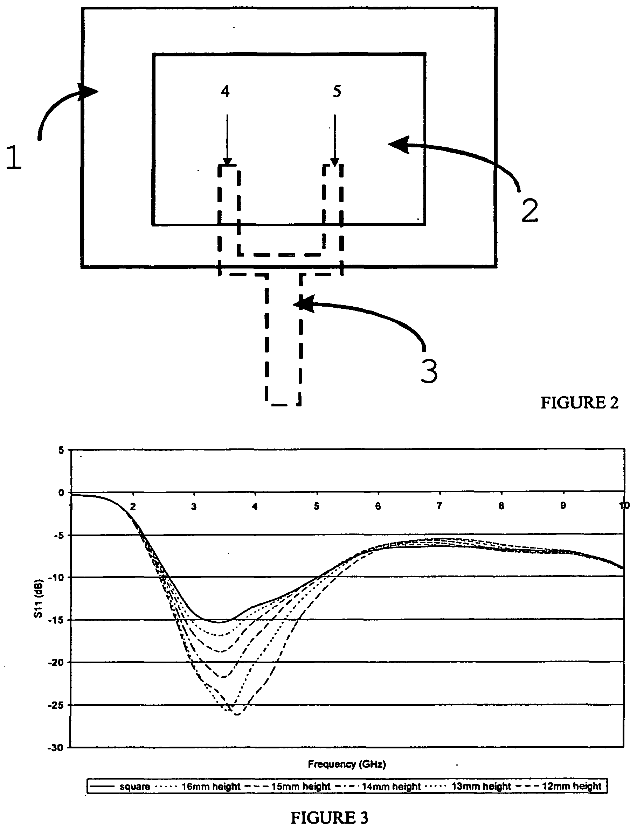

The antenna shown in Figures 1 and 2 comprises a slot 2 formed in a conductive element 1, the slot 2 having a rectangular external boundary defined by a substantially closed internal edge of the conductive element 1. The slot 2 is a continuous slot with no internal boundary, the boundary of the slot being completely defined by the internal edge of the conductive element 1.

A microstrip feed line 3 is spaced from the conductive element 1 as can be seen in Figure 1B, and the distal end of the line 3 is positioned at the geometric centre of the slot 2 as can be seen in Figure 1A. The feed line couples energy to and/or from the slot 2 in a known fashion.

The feed line 3 and conductive element 1 are mounted on opposite sides of a dielectric substrate (not shown).

In the variant shown in Figure 1c, the rear side of the slot 2 is substantially enclosed by walls 21,22,23 of a conducting material, which together define a cavity 20. In this case the slot 2 will only radiate into a half space in front of the slot (that is, away from the cavity 20).

The antenna shown in Figure 2 is similar to the antenna of Figures 1A and 1B, and the same reference numerals are used to indicate identical components. The microstrip feed line 3 splits into two parallel feed lines 4,5 each coupling energy to and/or from a respective part of the slot 2. The feed lines 4,5 are placed symmetrically with respect to the centerline of the slot. The rear side of the slot 2 in the embodiment of Figure 2 may also be substantially enclosed by walls of a conducting material as shown in Figure 1C.

The antennas shown in Figures 1 and 2 are configured to transmit radiation in a -10dB band of wavelengths including a minimum wavelength of 9.5mm, a maximum wavelength of 32mm and a centre wavelength of 21mm, and the slot 2 has a maximum dimension (that is, the diagonal) which is smaller than the centre wavelength.

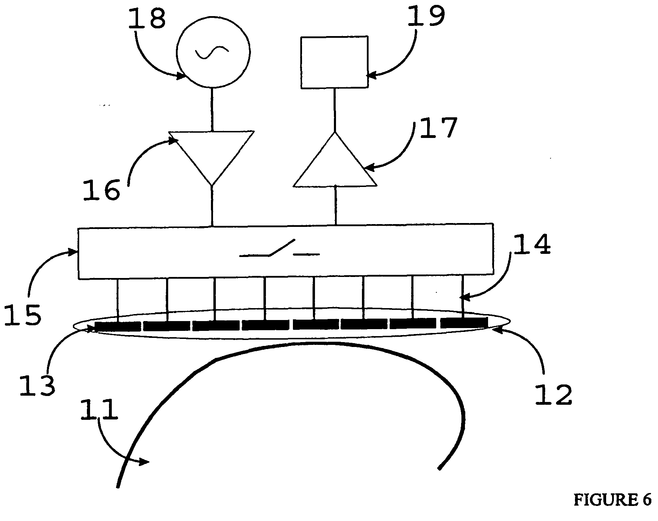

The antennas of Figures 1 and 2 are used in a real aperture synthetically organised radar system for breast cancer detection, shown in Figure 6. The system operates by employing a two-dimensional array 12 of N antennas (e.g. 13) close to, or in contact with, the breast 11. Each antenna in turn transmits a pulse and the received signal at each of the other antennas is input to circuitry 19. The circuitry 19 incorporates a receiver configured to detect the effect of the body on the passage of the electromagnetic energy by recording the received signals, and a processor configured to process the signals in order to generate an output indicative of the internal structure of the breast.

The pulse generator 18 and the circuitry 19 may be time-shared, by means of a switching matrix 15 as shown in Figure 6, as may any transmit or receive path amplification (16, 17). The mode of operation of the system of Figure 6 is operated as described in further detail in WO 2006/085052 A2, the contents of which is incorporated herein by reference.

The breast 11 has a relative permittivity of approximately 9. In order to minimise the size of the antenna the dielectric substrate chosen for the new antenna is RT/Duroid with a relative permittivity of εᵣ = 10.2. As the wavelength (λ) in the RT/Duroid substrate is smaller than that in the design of Sze et al., the dimensions of the slot and feed must be altered accordingly if the performance characteristics are to be kept. As the slot is to be in direct contact with the breast 11 and so no air dielectric interface exists, the ratio between the size of the antenna in Sze et al., and the size of the new antenna is the square root of 4.4/10.2 which is approximately 0.5. This allows the size of the slot 2 and feed fork (3,4,5) to be reduced by a factor of two using the RT/Duroid substrate. The width of the microstrip fork feed was recalculated for the new substrate permittivity. The resulting design is a square slot of 17x17mm.

The antenna is desired to be as compact as possible and as a result the effect of reducing the size of the slot is a major consideration. The effect that altering the z-dimension of the slot (the z axis being parallel to the feed lines 3,4,5) has on the performance of the antenna can be seen in Figure 3. Figure 3 shows that reducing the z-dimension of the slot has little effect on the -10dB bandwidth of the antenna although the return loss in the major null is improved by 10dB by decreasing the slot size. This is beneficial to the antenna design and allows significant size reduction of the slot down to 17x12mm (that is, 17mm in the x-direction and 12mm in the z direction).

It was found that the slot x-dimension was almost directly related to the antenna's lower bandwidth cut-off point. As the cut-off frequency is at a reasonable point no further investigation needs to be carried out.

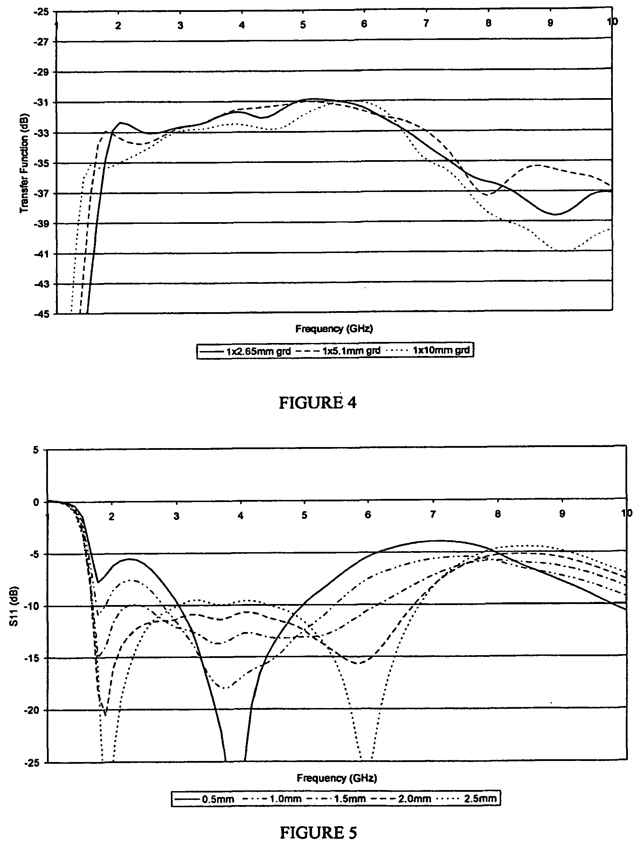

Figure 4 shows the bore sight transfer function of various antenna designs. These plots show that regardless of the geometry of the conductive element 1 (the "ground plane") the bore sight transfer function varies less than 10dB in the worst case (with the conductive element 1 being 2mm larger than the slot in the x direction, and 20mm larger in the z direction) and 6dB in the best case (the elements being 2mm larger in x and 10mm larger in z). The lack of variation and "flatness" of the transfer function across the frequency range of interest means that any signal transmitted by the antenna is likely to have minimal distortion. At angles away from bore sight the flattest transfer function is seen with a 5.1mm ground above and below the slot. A very small or large ground causes a greater variation in the transfer function away from bore sight. The most appropriate ground size will be 1 mm in the x-direction each side of the slot and 5 mm in the z direction on each of the top and bottom of the slot.

Varying the dimension of the conductive element 1 (the ground plane) in the *x*-direction has very little effect on the s11 of the antenna. The general trend in the transfer function data is that there is an improvement in the antenna's performance when the size of the ground plane in the *x*-direction is reduced. This is beneficial in minimising the size of the antenna and allows a 1mm ground each side of the slot to be used.

Figure 5 shows the variation in s11 with the size of the gap between the lower edge of the slot and the fork feed (the feed gap) for the optimised antenna configuration (17x12mm slot with a 1x5.1mm ground). This graph shows that as the feed gap increases in size from 0.5mm to 2.0mm the -10dB bandwidth increases from 2GHz (3-5GHz) to 5.5GHz (1.5-7GHz). Increasing the feed gap to values above 2mm results in no significant increase in - 10dB bandwidth, while the return loss increases in the 2.5GHz-0.5GHz frequency range, to above the -10dB level.

The transmission characteristics show a general improvement with increasing feed gap especially at higher frequencies. From these results the most effective feed gap will be 2mm. These results also highlight the large effect that even a small change in this parameter has over the performance of the antenna. As such much care must be taken when deciding the manufacturing tolerances of such an antenna.

Although the invention has been described above with reference to one or more preferred embodiments, it will be appreciated that various changes or modifications may be made without departing from the scope of the invention as defined in the appended claims.

In particular it is noted that further miniaturisation of the antenna is possible, and prototype designs with overall dimensions less than 10mm x 10mm have been successfully evaluated.

Also, the antenna may be replaced with a complementary structure having similar properties according to Babinet's principle. That is, the slot 2 may be replaced by a complementary patch of conductive material, and the microstrip feed lines 3,4,5 replaced by a complementary slot in a sheet of conductive material.

## Claims

1. A system for investigating the internal structure of a human or animal body, the system comprising:
one or more antennas for transmitting and/or receiving electromagnetic energy into or from the body;
a receiver (17) configured to detect the effect of the body on the passage of the electromagnetic energy by recording one or more signals; and
a processor (19) configured to process the signal(s) in order to generate an output indicative of the internal structure of the body,
**characterized in that** each antenna comprises a slot (2) formed in a conductive element, the slot having an external boundary defined by a substantially closed internal edge of the conductive element, wherein the antenna slot is a continuous slot with no internal boundary, the boundary of the slot being completely defined by the internal edge of the conductive element, and **in that** the slot of each antenna has a square or rectangular shape and a maximum dimension which is smaller than 30mm.

2. The system of claim 1 wherein each antenna further comprises one or more conductive elements (3,4,5) for coupling energy to and/or from the slot.

3. The system of claim 2 wherein the conductive elements comprise two or more conductive feed lines (4,5), each feed line coupling with a respective part of the slot.

4. The system of any preceding claim wherein one side of the slot is substantially enclosed by walls of a conducting material.

5. The system of any preceding claim wherein each antenna is configured to transmit and/or receive radiation in a range of wavelengths including a minimum, maximum and centre wavelength, and wherein the slot of each antenna has a maximum dimension which is smaller than the centre wavelength.

6. The system of any preceding claim wherein the slot of each antenna has a maximum dimension which is smaller than 18mm.

7. The system of any preceding claim, comprising two or more antennas.

8. The system of any preceding claim, further comprising means for sequentially driving the antennas.

9. The system of any preceding claim wherein the slot has an elongated shape.

10. A method of investigating the internal structure of a human or animal body (11) with the system of any preceding claim, the method comprising:
transmitting and/or receiving electromagnetic energy into and/or from the body with the antenna(s);
detecting the effect of the body on the passage of the electromagnetic energy by recording one or more signals; and
processing the signal(s) in order to generate an output indicative of the internal structure of the body.

## Patentansprüche

1. System zur Untersuchung der inneren Struktur eines menschlichen oder tierischen Körpers, wobei das System Folgendes umfasst:
eine oder mehrere Antennen zum Übertragen und/oder Empfangen von elektromagnetischer Energie in den oder aus dem Körper;
einen Empfänger (17), der ausgebildet ist, um die Wirkung des Körpers auf den Durchgang der elektromagnetischen Energie durch Aufzeichnen von einem oder mehreren Signalen zu detektieren; und
einen Prozessor (19), der ausgebildet ist, um das/die Signal(e) zu verarbeiten, um eine Ausgabe zu erzeugen, die die innere Struktur des Körpers anzeigt,
**dadurch gekennzeichnet, dass** jede Antenne einen Schlitz (2) umfasst, der in einem leitenden Element gebildet ist, wobei der Schlitz eine äußere Begrenzung aufweist, die durch eine im Wesentlichen geschlossene innere Kante des leitenden Elements definiert ist, wobei der Antennenschlitz ein durchgehender Schlitz mit keiner inneren Begrenzung ist, wobei die Begrenzung des Schlitzes vollständig durch die innere Kante des leitenden Elements definiert ist, und dass der Schlitz jeder Antenne eine quadratische oder rechteckige Form und eine maximale Abmessung aufweist, welche kleiner als 30 mm ist.

2. System nach Anspruch 1, wobei jede Antenne ferner ein oder mehrere leitende Elemente (3, 4, 5) zum Koppeln von Energie in den und/oder von dem Schlitz umfasst.

3. System nach Anspruch 2, wobei die leitenden Elemente zwei oder mehr leitende Zuführleitungen (4, 5) umfassen, wobei jede Zuführleitung mit einem jeweiligen Teil des Schlitzes gekoppelt ist.

4. System nach einem der vorherigen Ansprüche, wobei eine Seite des Schlitzes im Wesentlichen von Wänden eines leitenden Materials umschlossen ist.

5. System nach einem der vorherigen Ansprüche, wobei jede Antenne ausgebildet ist, um Strahlung in einem Bereich von Wellenlängen einschließlich einer Mindest-, Höchst- und Mittenwellenlänge zu übertragen und/oder zu empfangen, und wobei der Schlitz jeder Antenne eine maximale Abmessung aufweist, welche kleiner als die Mittenwellenlänge ist.

6. System nach einem der vorherigen Ansprüche, wobei der Schlitz jeder Antenne eine maximale Abmessung aufweist, welche kleiner als 18 mm ist.

7. System nach einem der vorherigen Ansprüche, umfassend zwei oder mehr Antennen.

8. System nach einem der vorherigen Ansprüche, ferner umfassend Mittel zum sequentiellen Steuern der Antennen.

9. System nach einem der vorherigen Ansprüche, wobei der Schlitz eine längliche Form aufweist.

10. Verfahren zur Untersuchung der inneren Struktur eines menschlichen oder tierischen Körpers (11) mit dem System nach einem der vorherigen Ansprüche, wobei das Verfahren Folgendes umfasst:
Übertragen und/oder Empfangen von elektromagnetischer Energie in den und/oder von dem Körper mit der/den Antenne(n);
Detektieren der Wirkung des Körpers auf den Durchgang der elektromagnetischen Energie durch Aufzeichnen von einem oder mehreren Signalen; und
Verarbeiten des/der Signals/Signale, um eine Ausgabe zu erzeugen, die die innere Struktur des Körpers anzeigt.

## Revendications

1. Système pour étudier la structure interne d'un corps humain ou animal, le système comprenant :
une ou plusieurs antennes pour transmettre et/ou recevoir de l'énergie électromagnétique vers ou à partir du corps ;
un récepteur (17) configuré pour détecter l'effet du corps sur le passage de l'énergie électromagnétique en enregistrant un ou plusieurs signaux ; et
un processeur (19) configuré pour traiter le signal ou les signaux afin de générer une sortie indicative de la structure interne du corps,
**caractérisé en ce que** chaque antenne comporte une fente (2) formée dans un élément conducteur, la fente ayant une limite externe définie par un bord interne sensiblement fermé de l'élément conducteur, dans lequel la fente d'antenne est une fente continue sans limite interne, la limite de la fente étant complètement définie par le bord interne de l'élément conducteur, et **en ce que** la fente de chaque antenne présente une forme carrée ou rectangulaire et une dimension maximale qui est inférieure à 30 mm.

2. Système selon la revendication 1, dans lequel chaque antenne comprend en outre un ou plusieurs éléments conducteurs (3, 4, 5) pour coupler de l'énergie vers et/ou depuis la fente.

3. Système selon la revendication 2, dans lequel les éléments conducteurs sont constitués de deux lignes d'alimentation conductrices (4, 5) ou plus, chaque ligne d'alimentation se couplant à une partie respective de la fente.

4. Système selon l'une quelconque des revendications précédentes, dans lequel un côté de la fente est sensiblement enfermé par des parois en un matériau conducteur.

5. Système selon l'une quelconque des revendications précédentes, dans lequel chaque antenne est configurée pour transmettre et/ou recevoir un rayonnement dans une plage de longueurs d'onde comprenant des longueurs d'onde minimale, maximale et centrale, et dans lequel la fente de chaque antenne a une dimension maximale qui est inférieure à celle de la longueur d'onde centrale.

6. Système selon l'une quelconque des revendications précédentes, dans lequel la fente de chaque antenne a une dimension maximale qui est inférieure à 18 mm.

7. Système selon l'une quelconque des revendications précédentes, comprenant deux antennes ou plus.

8. Système selon l'une quelconque des revendications précédentes, comprenant en outre des moyens pour entraîner les antennes séquentiellement.

9. Système selon l'une quelconque des revendications précédentes, dans lequel la fente a une forme allongée.

10. Procédé pour étudier la structure interne d'un corps humain ou animal (11) à l'aide du système selon l'une quelconque des revendications précédentes, le procédé comprenant les étapes consistant à :
transmettre et/ou recevoir de l'énergie électromagnétique dans et/ou à partir du corps à l'aide de la ou des antennes ;
détecter l'effet du corps sur le passage de l'énergie électromagnétique en enregistrant un ou plusieurs signaux ; et
traiter le ou les signaux afin de générer une sortie indicative de la structure interne du corps.
